# EUROPÄISCHE PATENTSCHRIFT

(11) **EP 0 791 033 B1**
(45) Veröffentlichungstag und Bekanntmachung des Hinweises auf die Patenterteilung: **01.07.1998**
(21) Anmeldenummer: 95939247.3
(22) Anmeldetag: 09.11.1995
(51) Int. Cl.: C08L 5/16, C08J 3/12, C08F 2/44

(54) **REDISPERGIERBARE POLYMERPULVER-ZUSAMMENSETZUNG ENTHALTEND CYCLODEXTRINE ODER CYCLODEXTRIN-DERIVATE**
REDISPERSABLE POLYMER POWDER COMPOSITIONS CONTAINING CYCLODEXTRIN OR CYCLODEXTRIN DERIVATIVES
COMPOSITION PULVERULENTE POLYMERE REDISPERSIBLE CONTENANT DE LA CYCLODEXTRINE OU DES DERIVES DE CYCLODEXTRINE

(30) Priorität: 10.11.1994 DE 4440236
(43) Veröffentlichungstag der Anmeldung: 27.08.1997
(73) Patentinhaber: WACKER-CHEMIE GMBH, D-81737 München (DE)
(72) Erfinder: HAAS, Wolfgang, D-84503 Altötting (DE); FIGGE, Reiner, D-84539 Ampfing (DE)
(74) Vertreter: Schuderer, Michael
(86) Internationale Anmeldenummer: EP9504412
(87) Internationale Veröffentlichungsnummer: WO9615187

(56) Entgegenhaltungen:
- EP-A- 0 134 451
- EP-A- 0 355 685
- EP-A- 0 536 597
- US-A- 5 147 907
- Dialog Information Services, file 351, DERWENT WPI, Dialog accession no. 003820382, WPI accession no. 83-816629/46, NICHIDEN KAGAKU KK; "Radical poly-merisation of monomer in presence of lipophilic cyclodextrin cpd. and radical polymerisation initiator"; & JP,A,58168603, 831005, 8346 (Basic), Die ganze Abstrakt

## Beschreibung

Die Erfindung betrifft redispergierbare Polymerpulver-Zusammensetzungen, welche Homo- oder Copolymerisate ethylenisch ungesättigter Monomere und Cyclodextrine oder Cyclodextrin-Derivate enthalten; Verfahren zu deren Herstellung sowie deren Verwendung. Weiter betrifft die Erfindung die als Zwischenprodukte erhältlichen, mit Cyclodextrinen oder Cyclodextrin-Derivaten stabilisierten, Polymerdispersionen.

Redispergierbare Pulver, die aus Dispersionen der verschiedensten Polymerisate hergestellt werden können, gewinnen für die unterschiedlichsten Anwendungen immer mehr an Bedeutung. Im allgemeinen wird bei der Herstellung dieser Pulver von mit Schutzkolloid und/oder Emulgator stabilisierten Dispersionen ausgegangen. Diese werden in bekannten Verfahren getrocknet und dabei oder danach mit Zusätzen gegen Verblokkung während der Lagerung geschützt. Als Schutzkolloide werden ,z.B., synthetisch hergestellte wasserlösliche Homo- und Copolymerisate oder Naturprodukte wie Cellulose- und Stärke-Derivate eingesetzt. Viele bekannte Dispersionen lassen sich jedoch trotz Zusatz von Schutzkolloiden nicht zu redispergierbaren Pulvern trocknen.

Aus der EP-A 134451 ist die Herstellung von redispergierbaren Polymerpulvern bekannt, die durch Trocknung von Polymerdispersionen erhältlich sind, welche durch Polymerisation in Gegenwart von Stärke und/oder Stärkederivaten hergestellt werden. Als Stärkederivate werden durch Hydrolyse von Stärke zugängliche Dextrine beschrieben.

In der EP-A 536597 (CA-A 2079726) wird die Herstellung wäßriger Polymerisatdispersionen in Gegenwart von, durch Hitzeeinwirkung auf Stärke zugänglichen, Röstdextrinen diskutiert. Darüberhinaus betrifft die Anmeldung die Herstellung von wäßrigen Polymerisatdispersionen in Gegenwart von, durch Hydrolyse in wäßriger Phase zugänglichen, Stärkeabbauprodukten mit bimodaler Molekulargewichtsverteilung. Ein weiterer Gegenstand ist die Herstellung von Polymerisatpulver durch Trocknung der genannten wäßrigen Polymerdispersionen.

Die EP-A 408099 (US-A 5147907) betrifft die Herstellung wäßriger Polymerdispersionen, wobei die Polymerisation in Gegenwart von Röstdextrinen mit einem Molekulargewicht > 5000 durchgeführt wird. Vom Einsatz niedermolekularer Stärkeabbauprodukte wird abgeraten.

Nachteilig bei allen genannten Verfahrensweisen ist, daß bei Verwendung von Stärkeabbauprodukten wie Dextrinen, welche durch saure Hydrolyse (Säuredextrine) oder durch Hitzebehandlung (Röstdextrine) erhältlich sind, keine definierten Verbindungen vorliegen, sondern ein Gemisch von mehr oder weniger langen, verzweigten oder unverzweigten Glucoseketten. Die Molekulargewichte können über einen relativ großen Bereich schwanken und sind nur in Bereichen angebbar. Als Folge der Schwankung ist das Polymerisationsergebnis bei Verwendung solcher Gemische nicht zuverlässig reproduzierbar; es kommt zu Schwankungen bezüglich der Produktqualität der Polymerdispersionen, die sich auch auf die Eigenschaften daraus hergestellter redispergierbarer Polymerpulver auswirken können. Viele Dispersionen lassen sich trotz Zusatz der Stärkeabbauprodukte als Schutzkolloid nicht zu redispergierbaren Pulvern trocknen. Die Pulvereigenschaften und die anwendungstechnischen Eigenschaften damit hergestellter Produkte unterliegen darüber hinaus starken Schwankungen, die zum einen von der verwendeten Rohstoffbasis, zum anderen von dem Hydrolyseverfahren beeinflußt werden.

Es bestand daher die Aufgabe redispergierbare Polymerpulver zur Verfügung zu stellen, deren anwendungstechnische Eigenschaften durch Verwendung von möglichst niedermolekularen Verbindungen, mit möglichst geringer Schwankungsbreite bezüglich deren Zusammensetzung, als Schutzkolloide zuverlässig einstellbar sind.

Gelöst wurde diese Aufgabe mit Polymerpulvern, welche Cyclodextrine oder Cyclodextrin-Derivate enthalten.

Aus der EP-A 355685 ist bekannt, Cyclodextrine als Schaumdämpfer in Polymerdispersionen einzusetzen. Die JP-A 58168603 (Derwent-Abstract AN 83-816629) beschreibt die Verwendung von lipophilen Cyclodextrin-Derivaten bei der Polymerisation in zweiphasigen Systemen aus Wasser und organischem Lösungsmittel.

Gegenstand der Erfindung sind redispergierbare Polymerpulver-Zusammensetzungen, welche Homo- oder Copolymerisate ethylenisch ungesättigter Monomere und Cyclodextrine oder Cyclodextrin-Derivate der allgemeinen Formel 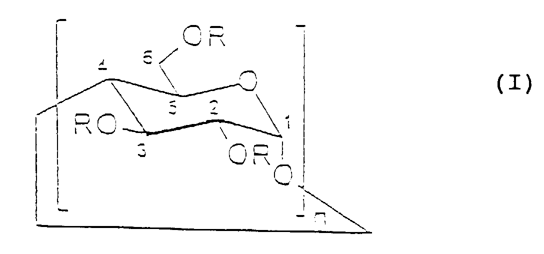 enthalten, wobei n = 6, 7 oder 8 und R gleich oder verschieden ist und die Bedeutung H oder R¹ hat, wobei R¹ gleich oder verschieden ist und die Bedeutung von gegebenenfalls substituierten C₁-C₄-Alkyl-, Hydroxy-C₁-C₄-Alkyl-, Carboxy-C₁-C₄-Alkyl-, C₂-C₄-Carboxyl-Reste hat.

Geeignete Homo- oder Copolymerisate enthalten ein oder mehrere Monomereinheiten aus der Gruppe der Vinylester von unverzweigten oder verzweigten Carbonsäuren mit 1 bis 18 C-Atomen, aus der Gruppe der Ester der Acrylsäure und Methacrylsäure mit unverzweigten oder verzweigten Alkoholen mit 1 bis 18 C-Atomen, aus der Gruppe der Vinylaromaten, aus der Gruppe der Vinylhalogenide und aus der Gruppe der Olefine.

Bevorzugte Vinylester sind Vinylacetat, Vinylpropionat, Vinylbutyrat, Vinyl-2-ethylhexanoat, Vinyllaurat, 1-Methylvinylacetat, Vinylpivalat und Vinylester von α-verzweigten Monocarbonsäuren mit 5 oder 9 bis 10 C-Atomen, beispielsweise VV5^{R}, VeoVa9^{R} oder VeoVa10^{R}. Besonders bevorzugt ist Vinylacetat.

Bevorzugte Methacrylsäureester oder Acrylsäureester sind Methylacrylat, Methylmethacrylat, Ethylacrylat, Ethylmethacrylat, Propylacrylat, Propylmethacrylat, n-Butylacrylat, n-Butylmethacrylat, iso-Butylacrylat, iso-Butylmethacrylat, t-Butylacrylat, t-Butylmethacrylat, 2-Ethylhexylacrylat, 2-Ethylhexylmethacrylat. Besonders bevorzugt sind Methylacrylat, Methylmethacrylat, n-Butylacrylat und 2-Ethylhexylacrylat.

Bevorzugte Vinylaromaten sind Styrol, α-Methylstyrol, o-Chlorstyrol oder Vinyltoluole.

Bevorzugte Vinylhalogenide sind Vinylchlorid und Vinylidenchlorid.

Bevorzugte Olefine sind Ethylen, Propylen, 1,3-Butadien, Isopren.

Die Vinylester-Copolymerisate können gegebenenfalls 1.0 bis 50 Gew%, bezogen auf das Gesamtgewicht des Copolymerisats, α-Olefine wie Ethylen oder Propylen und/oder Vinylaromaten wie Styrol und/oder Vinylhalogenide wie Vinylchlorid und/oder Acrylsäureester bzw. Methacrylsäureester von Alkoholen mit 1 bis 12 C-Atomen, wie Methylacrylat, Methylmethacrylat, Ethylacrylat, Ethylmethacrylat, Propylacrylat, Propylmethacrylat, n-Butylacrylat, n-Butylmethacrylat, 2-Ethylhexylacrylat und/oder ethylenisch ungesättigte Dicarbonsäureester bzw. deren Derivate wie Diisopropylfumarat, Di-t-butylfumarat, die Dimethyl-, Dibutyl- und Diethylester der Maleinsäure bzw. Fumarsäure, Maleinsäureanhydrid oder Acrylnitril enthalten. Die Auswahl aus den genannten Monomeren wird dabei vorzugsweise so getroffen, daß Copolymerisate mit einer Glasübergangstemperatur T_{g} von -30°C bis +40°C erhalten werden.

Die (Meth)acrylsäureester-Copolymerisate können gegebenenfalls 1.0 bis 50 Gew%, bezogen auf das Gesamtgewicht des Copolymerisats, α-Olefine wie Ethylen oder Propylen und/oder Vinylaromaten wie Styrol und/oder Vinylhalogenide wie Vinylchlorid und/oder ethylenisch ungesättigte Dicarbonsäureester bzw. deren Derivate wie Diisopropylfumarat, Di-t-butylfumarat, die Dimethyl-, Dibutyl- und Diethylester der Maleinsäure bzw. Fumarsäure, Maleinsäureanhydrid oder Acrylnitril enthalten. Die Auswahl aus den genannten Monomeren wird dabei vorzugsweise so getroffen, daß Copolymerisate mit einer Glasübergangstemperatur T_{g} von -30°C bis +40°C erhalten werden.

Gegebenenfalls enthalten die genannten Polymerisate noch 0.05 bis 30.0 Gew%, vorzugsweise 0.5 bis 15 Gew%, jeweils bezogen auf das Gesamtgewicht des Polymerisats, ein oder mehrere Hilfsmonomereinheiten zur Verbesserung der Wasserlöslichkeit, zur Vernetzung oder zur Modifikation der Haftungseigenschaften.

Geeignete Hilfsmonomere zur Verbesserung der Wasserlöslichkeit sind beispielsweise α,β-monoethylenisch ungesättigte Mono- und Dicarbonsäuren und deren Amide, wie Acrylsäure, Methacrylsäure, Maleinsäure, Fumarsäure, Itaconsäure, Acrylamid, Methacrylamid; ethylenisch ungesättigte Sulfonsäuren bzw. deren Salze, vorzugsweise Vinylsulfonsäure, 2-Acrylamido-propansulfonat und/oder N-Vinylpyrrolidon.

Vernetzend wirkende Monomereinheiten sind vorzugsweise zu 0.5 bis 5.0 Gew%, bezogen auf das Gesamtgewicht des Polymerisats, im Polymerisat enthalten. Beispiele hierfür sind N-Methylolacrylamid, N-Methylolmethacrylamid; N-(Alkoxymethyl)acrylamide oder N-(Alkoxymethyl)methacrylamide mit einem C₁- bis C₆-Alkylrest, wie N-(Isobutoxymethyl)-acrylamid (IBMA), N-(Isobutoxymethyl)-methacrylamid (IBMMA), N-(n-Butoxymethyl)-acrylamid (NBMA), N-(n-Butoxymethyl)-methacrylamid (NBMMA); mehrfach ethylenisch ungesättigten Comonomere wie Ethylenglycoldiacrylat, 1,3-Butylenglycoldiacrylat, 1,4-Butylenglycoldiacrylat, Propylenglycoldiacrylat, Divinyladipat, Divinylbenzol, Vinylmethacrylat, Vinylacrylat, Allylmethacrylat, Allylacrylat, Diallylmaleat, Diallylphthalat, Diallylfumarat, Methylenbisacrylamid, Cyclopentadienylacrylat oder Triallylcyanurat.

Zur Modifikation der Haftungseigenschaften geeignete Comonomereinheiten sind beispielsweise Methacrylsäure- und Acrylsäurehydroxyalkylester wie Hydroxyethyl-, Hydroxypropyl- oder Hydroxybutyl-acrylat oder -methacrylat sowie Verbindungen wie Diacetonacrylamid und Acetylacetoxyethylacrylat oder -methacrylat.

Bevorzugte Vinylester-Polymerisate enthalten als Monomereinheiten, jeweils bezogen auf das Gesamtgewicht des Polymerisats:
50 bis 100 Gew% Vinylester, insbesonders Vinylacetat;
50 bis 95 Gew% Vinylester, insbesonders Vinylacetat sowie 5 bis 50 Gew% α-Olefin, insbesonders Ethylen;
50 bis 75 Gew% Vinylacetat, 1 bis 30 Gew% Vinyllaurat oder Vinylester einer α-verzweigten Carbonsäure, insbesonders Versaticsäure-Vinylester, sowie 5 bis 40 Gew% Ethylen;
70 bis 99 Gew% Vinylacetat und 1 bis 30 Gew% Vinyllaurat oder Vinylester einer α-verzweigten Carbonsäure, insbesonders Versaticsäure-Vinylester;
70 bis 99 Gew% Vinylester, insbesonders Vinylacetat, und 1 bis 30 Gew% Acrylsäureester, insbesonders n-Butylacrylat oder 2-Ethylhexylacrylat;
50 bis 75 Gew% Vinylacetat, 1 bis 30 Gew% Acrylsäureester, insbesonders n-Butylacrylat oder 2-Ethylhexylacrylat, sowie 5 bis 40 Gew% Ethylen;
30 bis 75 Gew% Vinylacetat, 1 bis 30 Gew% Vinyllaurat oder Vinylester einer α-verzweigten Carbonsäure, insbesonders Versaticsäure-Vinylester, 1 bis 30 Gew% Acrylsäureester, insbesonders n-Butylacrylat oder 2-Ethylhexylacrylat, sowie 5 bis 40 Gew% Ethylen.

Bevorzugte (Meth)acrylsäureester-Polymerisate enthalten als Monomereinheiten, jeweils bezogen auf das Gesamtgewicht des Polymerisats:
35 bis 65 Gew% Methylmethacrylat, 65 bis 35 Gew% n-Butylacrylat und/oder 2-Ethylhexylacrylat;
35 bis 65 Gew% Styrol und 65 bis 35 Gew% n-Butylacrylat und/oder 2-Ethylhexylacrylat.

Die als bevorzugt genannten Vinylester-Polymerisate und (Meth)acrylsäureester-Polymerisate können darüberhinaus noch die vorher genannten Hilfsmonomere in den genannten Mengen enthalten. Die Angaben in Gew% für die Monomergehalte bei den genannten bevorzugten Vinylester- und (Meth)acrylsäureester-Polymerisate addieren sich jeweils auf 100 Gew%.

Geeignete Cyclodextrine sind α-Cyclodextrin (Cyclohexaamylose, Formel I: n=6, R=H)), β-Cyclodextrin (Cycloheptaamylose, Formel I: n=7, R=H), γ-Cyclodextrin (Cyclooctaamylose, Formel I: n=8, R=H) oder Gemische der genannten Cyclodextrine.

Die Cyclodextrine sind durch enzymatische Spaltung von Stärke mit Cyclodextringlykosyltransferasen (CGTasen) und anschließender Aufarbeitung der dabei erhaltenen Cyclodextringemische mittels chromatographischer Trennung oder Trennung mittels Komplexbildnern in reiner Form zugänglich. Derartige Verfahren sind beispielsweise in der DE-A 4324650 beschrieben. Darüberhinaus sind α-, β- und γ-Cyclodextrin im Handel erhältlich.

Beispiele für Cyclodextrin-Derivate sind aus der Gruppe der Cyclodextrin-Alkylether die Methylether, Ethylether oder Propylether von α-, β-, γ-Cyclodextrin. Beispiele aus der Gruppe der Hydroxyalkylether sind Hydroxyethyl-, Hydroxypropyl- und Dihydroxypropyl-Ether von α-, β- und γ-Cyclodextrin. Beispiele aus der Gruppe der Carboxyalkyl-Ether sind Carboxymethyl- und Carboxypropylether von α-, β- und γ-Cyclodextrin und deren Alkalisalze wie der Natrium-Carboxymethylether. Geeignete Cyclodextrinether sind auch Mischether von α-, β- und γ-Cyclodextrin, welche mindestens zwei unterschiedliche Gruppen der genannten Alkylether-, Hydroxyalkylether- oder Carboxyalkylether-Gruppen aufweisen. Beispiele für Cyclodextrinester sind die Essigsäure- (Acetyl-Cyclodextrine), Propionsäure-Ester (Propionyl-Cyclodextrine) von α-, β- und γ-Cyclodextrin. Beispiele für substituierte Cyclodextrin-Ether oder Cyclodextrin-Ester sind 2-Aminoethyl- oder 2-Chloracetyl-Cyclodextrine.

Bevorzugte Cyclodextrin-Derivate sind die Methylether, Hydroxypropylether, Carboxymethylether und die Essigsäureester von α-, β- und γ-Cyclodextrin.

Substituierte Cyclodextrine werden auch durch deren durchschnittlichen Substitutionsgrad (DS-Wert, average degree of substitution) charakterisiert. Der DS-Wert gibt an, wieviele Substituenten im Schnitt pro Anhydroglucose-Einheit an der 02-, 03- und/oder 06-Position gebunden sind. Die Bestimmung des DS-Wertes ist beispielsweise über ¹H-NMR-Sektroskopie möglich. Vorzugsweise beträgt der Substitutionsgrad der Cyclodextrinderivate von 0.5 bis 2.5.

Verfahren zur Herstellung der genannten Cyclodextrin-Derivate sind dem Fachmann bekannt und beispielsweise in der EP-A 146841 (US-A 4582900) und der US-A 3565887 beschrieben.

Vorzugsweise enthalten die Polymerpulver-Zusammensetzungen 0.3 bis 20 Gew% Cyclodextrin oder Cyclodextrin-Derivate, bezogen auf den Polymeranteil. Gegebenenfalls enthalten die Polymerpulver-Zusammensetzungen noch weitere Zusatzstoffe wie Verdüsungshilfen, Antischaummittel und/oder Antiblockmittel.

Ein weiterer Gegenstand der Erfindung ist ein Verfahren zur Herstellung der redispergierbaren Polymerpulver-Zusammensetzungen, welche Homo- oder Copolymerisate ethylenisch ungesättigter Monomere und Cyclodextrin oder Cyclodextrinderivate der allgemeinen Formel (I) enthalten, durch
a) Emulsionspolymerisation eines oder mehrerer ethylenisch ungesättigter Monomere mittels Radikalinitiatoren in wäßrigem Medium, in Gegenwart von Cyclodextrin oder Cyclodextrinderivaten der allgemeinen Formel (I), gegebenenfalls von Emulgator, und in Abwesenheit von Schutzkolloiden und
b) Trocknung der dabei erhaltenen wäßrigen Polymerdispersion, gegebenenfalls unter Zugabe von Antiblockmittel und/oder weiterer Zusatzstoffe.

Die Herstellung der wäßrigen Polymerdispersionen erfolgt nach dem Verfahren der wäßrigen Emulsionspolymerisation in Gegenwart von 0.3 bis 10.0 Gew%, bezogen auf das Gesamtgewicht der Monomere, Cyclodextrin oder Cyclodextrinderivaten der Formel (I). Die Polymerisationstemperatur beträgt in der Regel 35 - 95°C, bevorzugt 40 - 80°C. Als Polymerisationsmedium wird Wasser, gegebenenfalls ein Gemisch aus Wasser und aus mit Wasser gut mischbaren Lösungsmitteln wie Aceton oder Ethanol, eingesetzt. Vorzugsweise wird in Wasser polymerisiert. Die Polymerisation kann im Batchverfahren, wobei alle Komponenten im Reaktor vorgelegt werden, und im Dosierverfahren, wobei einzelne oder mehrere Komponenten während der Polymerisation zugeführt werden, durchgeführt werden. Mischtypen mit Vorlage und Dosierung werden bevorzugt. Die Dosierungen können separat (räumlich und zeitlich) durchgeführt werden oder die zu dosierenden Komponenten können alle oder teilweise voremulgiert dosiert werden. Die Cyclodextrine oder Cyclodextrinderivate können vorgelegt oder dosiert, oder auf Vorlage und Dosierung verteilt werden. Bevorzugt werden die Cyclodextrine oder Cyclodextrinderivate vorgelegt. Die Dosierungen können mit konstanter Rate und mit wechselnden Raten durchgeführt werden. Für den Einsatz des Initiatorsystems gelten die gleichen Maßgaben wie für den Einsatz der anderen Reaktionskomponenten.

Sollen beispielsweise gasförmige Reaktionskomponenten eingesetzt werden, kann die Emulsionspolymerisation auch unter erhöhtem Druck durchgeführt werden. Wird unter Druck gearbeitet, zum Beispiel beim Einsatz der Monomeren Vinylchlorid oder Ethylen, sind Drücke von 5 bar bis 80 bar bevorzugt. Entscheidend ist beispielsweise die Zielmenge an Ethylen, die einpolymerisiert werden soll.

Die erfindungsgemäß zu verwendenden Cyclodextrine bzw. Derivate der Formel (I) können sowohl allein als auch in Kombination mit Emulgatoren eingesetzt werden. Als begleitende Emulgatoren kommen anionische, kationische und auch nichtionische Emulgatoren in Betracht. Falls in Gegenwart von Emulgatoren polymerisiert wird, beträgt deren Menge vorzugsweise bis zu 4 Gew%, bezogen auf das Gesamtgewicht der Monomerphase. Vorzugsweise werden anionische und nichtionische Emulgatoren eingesetzt. Gebräuchliche Emulgatoren sind beispielsweise ethoxylierte Fettalkohole mit C₈-C₃₆-Alkylrest und einem Ethoxylierungsgrad (EO-Grad) von 3 bis 50; ethoxylierte Mono-, Di- und Trialkylphenole mit C₄-C₁₀-Alkylrest und einem EO-Grad von 3 bis 50; Alkalimetallsalze von Di-C₄-C₁₂-Alkylestern der Sulfobernsteinsäure. Geeignet sind auch Alkalimetall- und Ammoniumsalze von C₈-C₁₂-Alkylsulfaten, von ethoxylierten Alkanolen mit C₁₂-C₁₈-Alkylrest und einem EO-Grad von 3 bis 30, von ethoxylierten C₄-C₁₀-Alkylphenolen mit einem EO-Grad von 3 bis 50, von C₁₂-C₁₈-Alkylsulfonsäuren, von C₉-C₁₈-Alkylarylsulfonsäuren und von Sulfonaten ethoxylierter, linearer und verzweigter C₈-C₃₆-Alkylalkohole mit einem EO-Grad von 3 bis 50.

Die Initierung der Polymerisation erfolgt mit den für die Emulsionspolymerisation gebräuchlichen wasserlöslichen, thermischen Initiatoren oder Redox-Initiator-Kombinationen. Beispiele für thermische Initiatoren sind organische Peroxide wie tert.-Butylhydroperoxid, Cumylhydroperoxid, oder Peroxodisulfate wie Kaliumperoxodisulfat, Ammoniumperoxodisulfat, oder H₂O₂ oder Azoverbindungen wie Azodiisobutyronitril. Als Redox-Initiatoren verwendet man bevorzugt Wasserstoffperoxid, tert.-Butylhydroperoxid, Kaliumperoxodisulfat oder Ammoniumperoxodisulfat in Kombination mit Hydroxymethansulfinsäure, Ascorbinsäure oder Natriumsulfit als Reduktionsmittel. Zweckmäßigerweise wird die Reaktivität des Initiatorsystems durch Zusatz von Metallionen, die in mehreren Wertigkeitsstufen auftreten können, erhöht. Bevorzugt verwendet man Fe²⁺- oder Ce²⁺-Ionen. Die Initiatormenge beträgt vorzugsweise 0.01 bis 1.0 Gew%, bezogen auf das Gesamtgewicht der Monomerphase.

Zur Steuerung des Molekulargewichts können während der Polymerisation regelnde Substanzen eingesetzt werden. Sie werden üblicherweise in Mengen zwischen 0.01 bis 5.0 Gew%, bezogen auf die zu polymerisierenden Monomeren, eingesetzt und separat oder auch vorgemischt mit Reaktionskomponenten dosiert. Beispiele solcher Substanzen sind Dodecylmercaptan, Mercaptopropionsäure, Mercaptopropionsäuremethylester, Isopropanol und Acetaldehyd.

Die mit dem erfindungsgemäßen Verfahren erhältlichen wäßrigen Dispersionen haben einen Feststoffgehalt von 30 bis 75 Gew%, vorzugsweise von 40 bis 65 Gew%.

Ein weiterer Gegenstand der Erfindung sind wäßrige Polymerdispersionen mit einem Festgehalt von 30 bis 75 Gew%, welche Homo- oder Copolymerisate ethylenisch ungesättigter Monomere und Cyclodextrin oder Cyclodextrinderivate der allgemeinen Formel (I) enthalten, erhältlich durch Emulsionspolymerisation eines oder mehrerer ethylenisch ungesättigter Monomere mittels Radikal initiatoren in wäßrigem Medium, in Gegenwart von Cyclodextrin oder Cyclodextrinderivaten der allgemeinen Formel (I), gegebenenfalls von Emulgator, und in Abwesenheit von Schutzkolloiden.

Zur Herstellung der redispergierbaren Polymerpulver-Zusammensetzungen werden die wäßrigen Dispersionen schließlich getrocknet. Geeignete Trocknungsverfahren sind beispielsweise Sprühtrocknung, Gefriertrocknung, Walzentrocknung, Bandtrocknung oder Scheibentrocknung. Vorzugsweise werden die Dispersionen sprühgetrocknet oder gefriergetrocknet.

Am meisten bevorzugt ist die Sprühtrocknung der Dispersionen. Hierbei kann auf die bekannten Vorrichtungen, wie zum Beispiel Versprühen durch Ein-, Zwei- oder Mehrstoffdüsen oder mit einer rotierenden Scheibe, in einem gegebenenfalls erhitzten Trockengasstrom, vorzugsweise Luft, zurückgegriffen werden. Im allgemeinen werden Temperaturen über 250°C als Eintrittstemperatur des Trockengases nicht angewandt. Die Austrittstemperaturen des Trockengases liegen im allgemeinen im Bereich von 45 bis 100°C, bevorzugt 55 bis 90°C, je nach Anlage, Polymerzusammensetzung und gewünschtem Trocknungsgrad.

Zur Trocknung werden die Dispersionen auf einen Festgehalt von 10 bis 75 Gew%, vorzugsweise 30 bis 65 Gew% eingestellt. Der Festgehalt ist abhängig vom gewählten Trocknungsverfahren und von Art und Menge weiterer Zusatzstoffe, welche bei der Trocknung zugegeben werden. Für die bevorzugte Sprühtrocknung hat sich eine Viskosität des Gesamtsystems von bis zu 1000 mPa.s bewährt.

Beispielsweise können der Dispersion vor der Trocknung Verdüsungshilfen zugegeben werden. Die Zugabe erfolgt vorzugsweise in Form deren wäßriger Lösungen, in Mengen von vorzugsweise 5 bis 40 Gew%, insbesondere 5 bis 20 Gew%, bezogen auf das Polymerisat. Die optimale Menge richtet sich nach der Stabilisierung der Dispersion, der Glasübergangstemperatur des enthaltenen Polymerisats und den gewünschten Pulvereigenschaften.

Als Verdüsungshilfen eignen sich unter anderem, allein oder in Kombination, Cyclodextrine, Cyclodextrinderivate, abgebaute oder modifizierte Stärken, Stärkederivate, Cellulosederivate und wasserlösliche Polymere, insbesondere solche mit hohen Glasübergangstemperaturen von mindestens 50°C. Beispiele für derartige, vielfach handelsübliche Polymere sind: Vinylalkohol-Copolymere (Polyvinylalkohol) mit einem Hydrolysegrad von 85 bis 94 Mol% und einer Höppler-Viskosität, bestimmt in 4 %-iger Lösung, von 3 bis 25 mPa.s; vollverseifte Copolymere aus Vinylacetat und Alkylvinylestern mit einer Höppler-Viskosität von 1 bis 25 mPa.s; Vinylpyrrolidon-(Co)-Polymere; Ligninsulfonate; wasserlösliche sulfonatgruppenhaltige Kondensate aus Melamin und Formaldehyd oder Naphthalin und Formaldehyd; Phenolsulfonsäure-Formaldehyd-Kondensate; Polyacrylamide; Copolymerisate aus Styrol und Maleinsäure und/oder Itaconsäure und deren Ester; wasserlösliche Copolymere aus olefinisch ungesättigten Säuren und Alkenen; wasserlösliche Copolymere aus Monomeren wie Acrylsäure, Methacrylsäure, Itaconsäure, Fumarsäure, Methyl-, Ethyl-, Butyl-(meth)-acrylat, Ethylhexylacrylat, Decylacrylat, Hydroxyethyl-, Hydroxypropyl-(meth)-acrylat.

Bei der Verdüsung hat sich vielfach ein Gehalt von bis zu 1.5 Gew% Antischaummittel, bezogen auf das Basispolymerisat, als günstig erwiesen. Flüssige Antischaummittel werden normalerweise der Dispersion vor dem Trocknen zugesetzt, feste können in die trockene Dispersionspulverzusammensetzung eingemischt werden.

Zur Erhöhung der Lagerfähigkeit durch Verbesserung der Verblockungsstabilität, insbesonders bei Pulvern mit niedriger Glasübergangstemperatur, kann das erhaltene Pulver mit einem Antiblockmittel (Antibackmittel), vorzugsweise bis 30 Gew%, bezogen auf das Gesamtgewicht polymerer Bestandteile, versetzt werden. Dies erfolgt vorzugsweise, solange das Pulver noch fein verteilt ist, beispielsweise noch im Trockengas suspendiert ist. Insbesondere wird das Antiblockmittel getrennt aber gleichzeitig mit der Dispersion in die Trocknungsvorrichtung dosiert. Beispiele für Antiblockmittel sind fein gemahlene Aluminiumsilikate, Kieselgur, kolloidales Silicagel, pyrogene Kieselsäure, Fällungskieselsäure, Micro-Silica, Leichtspat, Kaolin, Talkum, Zemente, Diatomeenerde, Calciumcarbonat oder Magnesiumhydrosilikat.

Die erfindungsgemäßen Dispersionspulver eignen sich zur Verwendung in der Bauindustrie, insbesonders als Zusätze zu hydraulischen Bindemitteln wie Zement und Gips, insbesondere die Verwendung in Beton, Bauklebern, Mörteln, Spachtelmassen und Verlaufsmassen. Ferner eignen sich die Dispersionspulver als Bindemittel für Beschichtungen, Putze und Anstriche, insbesondere Anstrichfarben. Als Klebstoffe fur Holz, Papier, Textilien. Als Bindemittel bei der Papierherstellung. Als Bindemittel für Preßmassen und Formkörper sowie als Bindemittel zur Herstellung von Wirkstoffe enthaltenden, bevorzugt durch Direktverpressung hergestellten, Preßkörpern.

Die erfindungsgemäß hergestellten Dispersionspulver verbessern in Mörteln die Verarbeitungseigenschaften, reduzieren den Anmachwasserbedarf drastisch, verbessern deutlich die mechanischen Eigenschaften wie Haftung und Biegezugfestigkeit von erhärteten Mörteln.

Die nachfolgenden Beispiele und Vergleichsbeispiele dienen zur weiteren Erläuterung der Erfindung:

Zum Vergleich der Eigenschaften von Cyclodextrin(derivat)haltigen Dispersionspulvern mit solchen Pulvern ohne Cyclodextrin(derivat)-Gehalt wurden als Vergleichspulver Styrol/Butylacrylat- und Vinylacetat/Ethylen-Pulver ohne Zusatz von Cyclodextrinen oder Cyclodextrinderivaten hergestellt.

### Herstellung der Polymerdispersionen:

### Vergleichsbeispiel 1:

In einem Autoklaven wurden 101 kg vollentsalztes Wasser, 3.0 kg 20 %ige wässrige Lösung des Natriumsalzes von Dihexylsulfosuccinat und 1.65 kg 10 %ige Essigsäure vorgelegt. Der pH-Wert der Vorlage wurde mit 10 %iger Natronlauge auf 4.0 eingestellt. Unter Rühren wurden 22.5 kg Vinylacetat einemulgiert und auf 75°C aufgeheizt. Nach Erreichen des Temperaturgleichgewichtes drückte man bis 20 bar Ethylen auf. Anschließend begann man gleichzeitig mit dem Zudosieren von Lösungen von 630 g Kaliumperoxodisulfat in 20.4 kg Wasser und 316 g Hydroxymethansulfinsäure in 20.8 kg Wasser. Nach Reaktionsbeginn ließ man 203 kg Vinylacetat zulaufen. Weiter wurde eine Lösung aus 113 kg Wasser, 20 kg 30 %igem Natriumlaurylpolyglycolsulfat und 2.25 kg 50 %igem NatriumAcrylamido-2-methylpropansulfonat zudosiert. Nach beendetem Monomer- und Emulgatorzulauf wurde auch kein Ethylen mehr zugeführt und man dosierte das Initiatorsystem noch solange weiter bis der Festgehalt der Dispersion nicht mehr anstieg. Anschließend wurde gekühlt und entspannt. Zur vollständigen Auspolymerisation wurden nun 1.94 kg 10 %ige wäßrige tert.-Butylhydroperoxidlösung und 1.94 kg 10 %ige wäßrige Hydroxymethansulfinsäurelösung zugegeben.

Es resultierte ein Ethylen-Vinylacetat-Copolymerisat mit einem Ethylen-Gehalt von 15.0 Gew%, einem Vinylacetat-Gehalt von 84.6 Gew% und 0.4 Gew% AMPS. Die Dispersion enthielt keinen Grobanteil, und hatte einen Festgehalt von 49.8 Gew%, einen pH-Wert von 4.0 und eine mittlere Teilchengröße von 310 nm.

### Vergleichsbeispiel 2:

In einem Glasreaktor wurden 349 g vollentsalztes Wasser, 5.12 g 20 %ige wässrige Lösung des Natriumsalzes von Dihexylsulfosuccinat und 6.00 g 10 %ige Essigsäure vorgelegt. Der pH-Wert der Vorlage wurde mit 10 %iger Natronlauge auf 4.0 eingestellt. Unter Rühren wurden jeweils 41.0 g Styrol und Butylacrylat einemulgiert und auf 50°C aufgeheizt. In einem Dosierbehälter wurden 320 g Wasser, 55.6 g 30 %iges Natriumlaurylpolyglycolsulfat, 81.9 g 30 %iges Acrylamid, 369 g Styrol und 369 g Butylacrylat voremulgiert. Der pH-Wert der Voremulsion wurde mit 10 %iger Essigsäure auf 4.0 eingestellt. Anschließend begann man unter Rühren gleichzeitig mit dem Zudosieren von Lösungen von 12.9 g 40 %igem tert.-Butylhydroperoxid in 159 g Wasser und 8.82 g Hydroxymethansulfinsäure in 163 g Wasser. Nach Reaktionsbeginn wurde die Voremulsion gleichmäßig zudosiert. Nach beendetem Voremulsionszulauf dosierte man das Initiatorsystem noch solange weiter bis der Festgehalt der Dispersion nicht mehr anstieg. Anschließend wurde gekühlt. Zur vollständigen Auspolymerisation wurden nun 5.3 ml 10 %ige wäßrige tert.-Butylhydroperoxidlösung und 4.3 ml 10 %ige wäßrige Hydroxymethansulfinsäurelösung zugegeben.
Es resultierte ein Styrol-Butylacrylat-Copolymerisat mit einem Styrol-Gehalt von 48.5 Gew%, einem Butylacrylat-Gehalt von 48.5 Gew% und 3.0 Gew% Acrylamid. Die Dispersion enthielt keinen Grobanteil, und hatte einen Festgehalt von 49.0 Gew%, einen pH-Wert von 3.5 und eine mittlere Teilchengröße von 210 nm.

### Beispiel 1:

In einem Polymerisationskessel wurden 17.0 kg vollentsalztes Wasser, 923 g 20 %ige wässrige Lösung des Natriumsalzes von Dihexylsulfosuccinat, 270 g 10 %ige Essigsäure und 1.85 kg α-Cyclodextrin vorgelegt. Der pH-Wert der Vorlage wurde mit 10 %iger Natronlauge auf 4.1 eingestellt. Unter Rühren wurden jeweils 1.85 kg Styrol und Butylacrylat einemulgiert und auf 50°C aufgeheizt. In einem Dosierbehälter wurden 14.4 kg Wasser, 2.46 kg 30 %iges Natriumlaurylpolyglycolsulfat, 3.69 kg 30 %iges Acrylamid, 16.6 kg Styrol und 16.6 kg Butylacrylat voremulgiert. Der pH-Wert der Voremulsion wurde mit 10 %iger Essigsäure auf 4.1 eingestellt. Anschließend begann man unter Rühren gleichzeitig mit dem Zudosieren von Lösungen von 581 g 40 %igem tert.-Butylhydroperoxid in 7.17 kg Wasser und 399 g Hydroxymethansulfinsäure in 7.39 kg Wasser. Nach dem Start dieser Dosierungen wurde die Voremulsion zudosiert. Nach beendetem Voremulsionszulauf dosierte man das Initiatorsystem noch solange weiter bis der Festgehalt der Dispersion nicht mehr anstieg. Anschließend wurde gekühlt. Zur vollständigen Auspolymerisation wurden nun 240 ml 10 %ige wäßrige tert.-Butylhydroperoxidlösung und 240 g 10 %ige wäßrige Hydroxymethansulfinsäurelösung zugegeben.
Es resultierte ein Styrol-Butylacrylat-Copolymerisat mit einem Styrol-Gehalt von 48.5 Gew%, einem Butylacrylat-Gehalt von 48.5 Gew% und 3.0 Gew% Acrylamid. Die Dispersion enthielt kaum Grobanteil, und hatte einen Festgehalt von 44.6 Gew%, einen pH-Wert von 3.7 und eine mittlere Teilchengröße von 350 nm.

### Beispiel 2:

Es wurde analog Beispiel 1 vorgegangen mit dem Unterschied, daß als Cyclodextrin 1.85 kg β-Cyclodextrin verwendet wurde.
Es resultierte ein Styrol-Butylacrylat-Copolymerisat mit einem Styrol-Gehalt von 48.5 Gew%, einem Butylacrylat-Gehalt von 48.5 Gew% und 3.0 Gew% Acrylamid. Die Dispersion enthielt kaum Grobanteil, und hatte einen Festgehalt von 48.2 Gew%, einen pH-Wert von 4.4 und eine mittlere Teilchengröße von 300 nm.

### Beispiel 3:

Es wurde analog Beispiel 1 vorgegangen mit dem Unterschied, daß als Cyclodextrin 1.85 kg γ-Cyclodextrin verwendet wurde.
Es resultierte ein Styrol-Butylacrylat-Copolymerisat mit einem Styrol-Gehalt von 48.5 Gew%, einem Butylacrylat-Gehalt von 48.5 Gew% und 3.0 Gew% Acrylamid. Die Dispersion enthielt kaum Grobanteil, und hatte einen Festgehalt von 46.7 Gew%, einen pH-Wert von 3.7 und eine mittlere Teilchengröße von 260 nm.

### Beispiel 4:

Es wurde analog Beispiel 1 vorgegangen mit dem Unterschied, daß als Cyclodextrin 1.85 kg Acetyl-β-Cyclodextrin mit einem Substitutionsgrad von DS = 1.0 verwendet wurde.
Es resultierte ein Styrol-Butylacrylat-Copolymerisat mit einem Styrol-Gehalt von 48.5 Gew%, einem Butylacrylat-Gehalt von 48.5 Gew% und 3.0 Gew% Acrylamid. Die Dispersion enthielt kaum Grobanteil, und hatte einen Festgehalt von 46.2 Gew%, einen pH-Wert von 3.3 und eine mittlere Teilchengröße von 290 nm.

### Beispiel 5:

Es wurde analog Beispiel 1 vorgegangen mit dem Unterschied, daß als Cyclodextrin 1.85 kg Hydroxypropyl-β-Cyclodextrin mit einem Substitutionsgrad von DS = 0.9 verwendet wurde.
Es resultierte ein Styrol-Butylacrylat-Copolymerisat mit einem Styrol-Gehalt von 48.5 Gew%, einem Butylacrylat-Gehalt von 48.5 Gew% und 3.0 Gew% Acrylamid. Die Dispersion enthielt kaum Grobanteil, und hatte einen Festgehalt von 45.7 Gew%, einen pH-Wert von 3.4 und eine mittlere Teilchengröße von 300 nm.

### Beispiel 6:

Es wurde analog Beispiel 5 vorgegangen mit dem Unterschied, daß 0.75 kg Hydroxypropyl-β-Cyclodextrin mit einem Substitutionsgrad von DS = 0.9 verwendet wurde.
Es resultierte ein Styrol-Butylacrylat-Copolymerisat mit einem Styrol-Gehalt von 48.5 Gew%, einem Butylacrylat-Gehalt von 48.5 Gew% und 3.0 Gew% Acrylamid. Die Dispersion enthielt kaum Grobanteil, und hatte einen Festgehalt von 46.9 Gew%, einen pH-Wert von 4.0 und eine mittlere Teilchengröße von 1700 nm.

### Beispiel 7:

Es wurde analog Beispiel 1 vorgegangen mit dem Unterschied, daß als Cyclodextrin 1.85 kg Methyl-β-Cyclodextrin mit einem Substitutionsgrad von DS = 0.6 verwendet wurde.
Es resultierte ein Styrol-Butylacrylat-Copolymerisat mit einem Styrol-Gehalt von 48.5 Gew%, einem Butylacrylat-Gehalt von 48.5 Gew% und 3.0 Gew% Acrylamid. Die Dispersion enthielt kaum Grobanteil, und hatte einen Festgehalt von 44.8 Gew%, einen pH-Wert von 4.1 und eine mittlere Teilchengröße von 2000 nm.

### Beispiel 8:

Es wurde analog Beispiel 1 vorgegangen mit dem Unterschied, daß als Cyclodextrin 1.85 kg Carboxymethyl-β-Cyclodextrin mit einem Substitutionsgrad von DS = 0.3 verwendet wurde.
Es resultierte ein Styrol-Butylacrylat-Copolymerisat mit einem Styrol-Gehalt von 48.5 Gew%, einem Butylacrylat-Gehalt von 48.5 Gew% und 3.0 Gew% Acrylamid. Die Dispersion enthielt kaum Grobanteil, und hatte einen Festgehalt von 43.2 Gew%, einen pH-Wert von 4.2 und eine mittlere Teilchengröße von 300 nm.

### Beispiel 9:

In einem Druckreaktor wurden 2580 g vollentsalztes Wasser, 127 g 20 %ige wäßrige Lösung des Natriumsalzes von Dihexylsulfosuccinat, 46.5 ml 10 %ige Essigsäure und 317 g Acetyl-β-Cyclodextrin (Substitutionsgrad 1.0) (Ac-β-CD, DS = 1.0) vorgelegt. Der pH-Wert der Vorlage betrug 4.0. Unter Rühren wurden 635 g Vinylacetat einemulgiert und auf 75°C aufgeheizt. Nach Erreichen des Temperaturgleichgewichtes wurden bis 25 bar Ethylen aufgedrückt. Anschließend begann man gleichzeitig mit dem Zudosieren von Lösungen von 47.7 g Kaliumperoxodisulfat in 1540 g Wasser und 23.8 g Hydroxymethansulfinsäure in 1560 g Wasser. Nach Reaktionsbeginn wurden 5710 g Vinylacetat und eine Lösung von 265 g 30 %igem Natriumlaurylpolyglycolsulfat, 79.4 g 50 %igem Natrium-Acrylamido-2-methylpropansulfonat in 3960 g Wasser zudosiert. Nach beendetem Monomer- und Emulgatorzulauf wurde auch kein Ethylen mehr zugeführt und man dosierte das Initiatorsystem noch solange weiter bis der Festgehalt der Dispersion nicht mehr anstieg. Anschließend wurde gekühlt und entspannt. Zur vollständigen Auspolymerisation wurden nun 55 g 10 %ige wäßrige tert.-Butylhydroperoxidlösung und 55 g 10 %ige wäßrige Hydroxymethansulfinsäurelösung zugegeben.
Es resultierte ein Ethylen-Vinylacetat-Copolymerisat mit einem Ethylen-Gehalt von 15 Gew%, einem Vinylacetat-Gehalt von 84.6 Gew% und 0.4 Gew% AMPS. Die Dispersion enthielt keinen Grobanteil, und hatte einen Festgehalt von 50.5 Gew%, einen pH-Wert von 3.2 und eine mittlere Teilchengröße von 570 nm.

### Beispiel 10:

Es wurde analog Beispiel 9 vorgegangen mit dem Unterschied, daß als Cyclodextrin 131 g Hydroxypropyl-β-Cyclodextrin (DS = 0.9) verwendet wurde.
Es resultierte ein Ethylen-Vinylacetat-Copolymerisat mit einem Ethylen-Gehalt von 12.0 Gew%, einem Vinylacetat-Gehalt von 87.6 Gew% und 0.4 Gew% AMPS. Die Dispersion enthielt keinen Grobanteil, und hatte einen Festgehalt von 55 Gew%, einen pH-Wert von 3.0 und eine mittlere Teilchengröße von 260 nm.

### Beispiel 11:

Es wurde analog Beispiel 9 vorgegangen mit dem Unterschied, daß als Cyclodextrin 355 g Hydroxypropyl-β-Cyclodextrin (DS = 0.9) verwendet wurde.
Es resultierte ein Ethylen-Vinylacetat-Copolymerisat mit einem Ethylen-Gehalt von 12.0 Gew%, einem Vinylacetat-Gehalt von 87.6 Gew% und 0.4 Gew% AMPS. Die Dispersion enthielt keinen Grobanteil, und hatte einen Festgehalt von 49.6 Gew%, einen pH-Wert von 3.0 und eine mittlere Teilchengröße von 500 nm.

### Beispiel 12:

Es wurde analog Beispiel 9 vorgegangen mit dem Unterschied, daß als Cyclodextrin 131 g Methyl-β-Cyclodextrin (DS = 0.6) verwendet wurde.
Es resultierte ein Ethylen-Vinylacetat-Copolymerisat mit einem Ethylen-Gehalt von 9.0 Gew%, einem Vinylacetat-Gehalt von 90.5 Gew% und 0.5 Gew% AMPS. Die Dispersion enthielt keinen Grobanteil, und hatte einen Festgehalt von 52.3 Gew%, einen pH-Wert von 4.9 und eine mittlere Teilchengröße von 770 nm.

### Beispiel 13:

Es wurde analog Beispiel 9 vorgegangen mit dem Unterschied, daß als Cyclodextrin 218 g Methyl-β-Cyclodextrin (DS = 1.8) verwendet wurde.
Es resultierte ein Ethylen-Vinylacetat-Copolymerisat mit einem Ethylen-Gehalt von 15.0 Gew%, einem Vinylacetat-Gehalt von 84.6 Gew% und 0.4 Gew% AMPS. Die Dispersion enthielt keinen Grobanteil, und hatte einen Festgehalt von 50.9 Gew%, einen pH-Wert von 2.9 und eine mittlere Teilchengröße von 500 nm.

### Beispiel 14:

In einem Druckreaktor wurden 2480 g vollentsalztes Wasser, 143 g 20 %ige wässrige Lösung des Natriumsalzes von Dihexylsulfosuccinat und 52.3 ml 10 %ige Essigsäure vorgelegt. Der pH-Wert der Vorlage wurde mit 10 %iger Natronlauge auf 4.0 eingestellt. Unter Rühren wurden 714 g Vinylacetat einemulgiert und auf 75°C aufgeheizt. Nach Erreichen des Temperaturgleichgewichtes wurden bis 25 bar Ethylen aufgedrückt. Anschließend begann man gleichzeitig mit dem Zudosieren von Lösungen von 53,7 g Kaliumperoxodisulfat in 1730 g Wasser und 26.8 g Hydroxymethansulfinsäure in 1760 g Wasser. Nach Reaktionsbeginn wurden 6430 g Vinylacetat und eine Lösung von 714 g 30 %igem Natriumlaurylpolyglycolsulfat, 71.4 g 50 %igem Natrium-Acrylamido-2-methylpropansulfonat und 143 g Acetyl-β-Cyclodextrin (DS = 1.0) in 3570 g Wasser kontinuierlich zudosiert. Nach beendetem Monomer- und Emulgatorzulauf wurde auch kein Ethylen mehr zugeführt und man dosierte das Initiatorsystem noch solange weiter bis der Festgehalt der Dispersion nicht mehr anstieg. Anschließend wurde gekühlt und entspannt. Zur vollständigen Auspolymerisation wurden nun 61 ml 10 %ige wäßrige tert.-Butylhydroperoxidlösung und 61 ml 10 %ige wäßrige Hydroxymethansulfinsäurelösung zugegeben.
Es resultierte ein Ethylen-Vinylacetat-Copolymerisat mit einem Ethylen-Gehalt von 9.0 Gew%, einem Vinylacetat-Gehalt von 90.5 Gew% und 0.5 Gew% AMPS. Die Dispersion enthielt kaum Grobanteil, und hatte einen Festgehalt von 51.7 Gew.%, einen pH-Wert von 3.3 und eine mittlere Teilchengröße von 240 nm.

### Beispiel 15:

Beispiel 15 wurde analog Beispiel 14 durchgeführt, wobei statt 143 g Acetyl-β-Cyclodextrin 348 g Acetyl-β-Cyclodextrin eingesetzt wurden.
Es resultierte ein Ethylen-Vinylacetat-Copolymerisat mit einem Ethylen-Gehalt von 15.0 Gew%, einem Vinylacetat-Gehalt von 84.6 Gew% und 0.4 Gew% AMPS. Die Dispersion enthielt kaum Grobanteil, und hatte einen Festgehalt von 50.4 Gew.%, einen pH-Wert von 3.1 und eine mittlere Teilchengröße von 310 nm.

### Beispiel 16:

In einem Glasreaktor wurden 341 g vollentsalztes Wasser, 18.5 g 20 %ige wässrige Lösung des Natriumsalzes von Dihexylsulfosuccinat und 5.41 g 10 %ige Essigsäure vorgelegt. Der pH-Wert der Vorlage wurde mit 10 %iger Natronlauge auf 4.0 eingestellt. Unter Rühren wurden jeweils 36.9 g Styrol und Butylacrylat einemulgiert und auf 50°C aufgeheizt. In einem Dosierbehälter wurden 288 g Wasser, 45.2 g 30 %iges Natriumlaurylpolyglycolsulfat, 73.8 g 30 %iges Acrylamid, 332 g Styrol, 332 g Butylacrylat und 36.9 g Acetyl-β-Cyclodextrin (DS = 1.0) voremulgiert. Der pH-Wert der Voremulsion wurde mit 10 %iger Essigsäure auf 4.0 eingestellt. Anschließend begann man unter Rühren gleichzeitig mit dem Zudosieren von Lösungen von 11.6 g 40 %igem tert.-Butylhydroperoxid in 144 g Wasser und 7.95 g Hydroxymethansulfinsäure in 148 g Wasser. Nach Reaktionsbeginn wurde die Voremulsion gleichmäßig zudosiert. Nach beendetem Voremulsionszulauf dosierte man das Initiatorsystem noch solange weiter bis der Festgehalt der Dispersion nicht mehr anstieg. Anschließend wurde gekühlt. Zur vollständigen Auspolymerisation wurden nun 4.8 ml 10 %ige wäßrige tert.-Butylhydroperoxidlösung und 4.8 g 10 %ige wäßrige Hydroxymethansulfinsäurelösung zugegeben. Es resultierte ein Styrol-Butylacrylat-Copolymerisat mit einein Styrol-Gehalt von 48.5 Gew%, einem Butylacrylat-Gehalt von 48.5 Gew% und 3.0 Gew% Acrylamid. Die Dispersion enthielt kaum Grobanteil, und hatte einen Festgehalt von 48.2 Gew%, einen pH-Wert von 3.3 und eine mittlere Teilchengröße von 220 nm.

### Beispiel 17:

Beispiel 17 wurde analog Beispiel 16 durchgeführt, wobei statt 36.9 g Acetyl-β-Cyclodextrin 15.2 g Acetyl-β-Cyclodextrin eingesetzt wurden.
Es resultierte ein Styrol-Butylacrylat-Copolymerisat mit einem Styrol-Gehalt von 48.5 Gew%, einem Butylacrylat-Gehalt von 48.5 Gew% und 3.0 Gew% Acrylamid. Die Dispersion enthielt kaum Grobanteil, und hatte einen Festgehalt von 48.0 Gew%, einen pH-Wert von 3.4 und eine mittlere Teilchengröße von 250 nm.

### Beispiel 18:

In einem Glasreaktor wurden 2.38 kg vollentsalztes Wasser, 129 g 20 %ige wässrige Lösung des Natriumsalzes von Dihexylsulfosuccinat, 37.9 g 10 %ige Essigsäure und 259 g Hydroxypropyl-β-Cyclodextrin (DS = 0.9) vorgelegt. Der pH-Wert der Vorlage wurde mit 10 %iger Natronlauge auf 4.0 eingestellt. Unter Rühren wurden 228 g Styrol und 290 g Butylacrylat einemulgiert und auf 50°C aufgeheizt. In einem Dosierbehälter wurden 2.02 kg Wasser, 345 g 30 %iges Natriumlaurylpolyglycolsulfat, 517 g 30 %iges Acrylamid, 2.05 kg Styrol und 2.61 kg Butylacrylat vor emulgiert. Der pH-Wert der Voremulsion wurde mit 10 %iger Essigsäure auf 4.0 eingestellt. Anschließend begann man unter Rühren gleichzeitig mit dem Zudosieren von Lösungen von 81.6 g 40 %igem tert.-Butylhydroperoxid in 1.00 kg Wasser und 55.7 g Hydroxymethansulfinsäure in 1.03 kg Wasser. Nach Reaktionsbeginn wurde die Voremulsion gleichmäßig zudosiert. Nach beendetem Voremulsionszulauf dosierte man das Initiatorsystem noch solange weiter bis der Festgehalt der Dispersion nicht mehr anstieg. Anschließend wurde gekühlt. Zur vollständigen Auspolymerisation wurden nun 34 g 10 %ige wäßrige tert.-Butylhydroperoxidlösung und 34 g 10 %ige wäßrige Hydroxymethansulfinsäurelösung zugegeben.
Es resultierte ein Styrol-Butylacrylat-Copolymerisat mit einem Styrol-Gehalt von 42.7 Gew%, einem Butylacrylat-Gehalt von 54.4 Gew% und 2.9 Gew% Acrylamid. Die Glasübergangstemperatur betrug 10°C. Die Dispersion enthielt kaum Grobanteil, und hatte einen Festgehalt von 44.2 Gew%, einen pH-Wert von 3.3 und eine mittlere Teilchengröße von 280 nm.

### Beispiel 19:

Es wurde analog Beispiel 18 vorgegangen, mit dem Unterschied, daß die Vorlage 212 g Styrol und 305 g Butylacrylat enthielt und 1.91 kg Styrol zusammen mit 2.75 kg Butylacrylat als Voremulsion zudosiert wurden.
Es resultierte ein Styrol-Butylacrylat-Copolymerisat mit einem Styrol-Gehalt von 39.8 Gew%, einem Butylacrylat-Gehalt von 57.3 Gew% und 2.9 Gew% Acrylamid. Die Glasübergangstemperatur betrug 5°C. Die Dispersion enthielt kaum Grobanteil, und hatte einen Festgehalt von 45.2 Gew%, einen pH-Wert von 3.2 und eine mittlere Teilchengröße von 390 nm.

### Beispiel 20:

Es wurde analog Beispiel 18 vorgegangen, mit dem Unterschied, daß die Vorlage 219 g Styrol und 290 g Ethylhexylacrylat enthielt und 1.97 kg Styrol zusammen mit 2.61 kg Ethylhexylacrylat als Voremulsion zudosiert wurden.
Es resultierte ein Styrol-Ethylhexylacrylat-Copolymerisat mit einem Styrol-Gehalt von 41.7 Gew%, einem Ethylhexylacrylat-Gehalt von 55.3 Gew% und 3.0 Gew% Acrylamid. Die Glasübergangstemperatur betrug -5°C. Die Dispersion enthielt kaum Grobanteil, und hatte einen Festgehalt von 44.9 Gew%, einen pH-Wert von 3.6 und eine mittlere Teilchengröße von 430 nm.

### Beispiel 21:

In einem Polymerisationskessel wurden 17.0 kg vollentsalztes Wasser, 923 g 20 %ige wässrige Lösung des Natriumsalzes von Dihexylsulfosuccinat, 270 g 10 %ige Essigsäure und 1.85 kg Hydroxypropyl-β-Cyclodextrin (DS = 0.9) vorgelegt. Der pH-Wert der Vorlage wurde mit 10 %iger Natronlauge auf 4.1 eingestellt. Unter Rühren wurden jeweils 1.85 kg Styrol und Butylacrylat einemulgiert und auf 50°C aufgeheizt. In einem Dosierbehälter wurden 14.4 kg Wasser, 2.46 kg 30 %iges Natriumlaurylpolyglycolsulfat, 16.6 kg Styrol und 16.6 kg Butylacrylat voremulgiert. Der pH-Wert der Voremulsion wurde mit 10 %iger Essigsäure auf 4.1 eingestellt. Anschließend begann man unter Rühren gleichzeitig mit dem Zudosieren von Lösungen von 581 g 40 %igem tert.-Butylhydroperoxid in 7.17 kg Wasser und 399 g Hydroxymethansulfinsäure in 7.39 kg Wasser. Nach dem Start dieser Dosierungen wurde die Voremulsion gleichmäßig zudosiert. Nach beendetem Voremulsionszulauf dosierte man das Initiatorsystem noch solange weiter bis der Festgehalt der Dispersion nicht mehr anstieg. Anschließend wurde gekühlt. Zur vollständigen Auspolymerisation wurden nun 240 ml 10 %ige wäßrige tert.-Butylhydroperoxidlösung und 240 g 10 %ige wäßrige Hydroxymethansulfinsäurelösung zugegeben.
Es resultierte ein Styrol-Butylacrylat-Copolymerisat mit einem Styrol-Gehalt von 50.0 Gew% und einem Butylacrylat-Gehalt von 50.0 Gew%. Die Dispersion enthielt kaum Grobanteil, und hatte einen Festgehalt von 45.7 Gew%, einen pH-Wert von 3.4 und eine mittlere Teilchengröße von 300 nm.

### Beispiel 22:

In einem Laborautoklaven wurden 264 g vollentsalztes Wasser, 15.2 g 20 %ige wässrige Lösung des Natriumsalzes von Dihexylsulfosuccinat und 5.57 ml 10 %ige Essigsäure vorgelegt. Der pH-Wert der Vorlage wurde mit 10 %iger Natronlauge auf 4.0 eingestellt. Unter Rühren wurden 76 g Vinylacetat einemulgiert und auf 75°C aufgeheizt. Nach Erreichen des Temperaturgleichgewichtes wurden bis 25 bar Ethylen aufgedrückt. In einem Voremulsionsgefäß emulgiert man 380 g Wasser, 15.2 g Acetyl-β-Cyclodextrin (DS = 1.0) , 7.6 g 50 %iges Natrium-Acrylamido-2-methylpropansulfonat, 76.0 g 30 %iges Natriumlaurylpolyglycolsulfat und 684 g Vinylacetat. Anschließend begann man unter Rühren gleichzeitig mit dem Zudosieren von Lösungen von 6.65 g Kaliumperoxodisulfat in 215 g Wasser und 3.33 g Hydroxymethansulfinsäure in 218 g Wasser. Nach Reaktionsbeginn wurde die Voremulsion kontinuierlich zudosiert. Während dieser Zeit wurde der Ethylendruck auf 25 bar gehalten. Danach wurde die Ethylenzufuhr geschlossen und man dosierte das Initiatorsystem noch solange weiter bis der Festgehalt der Dispersion nicht mehr anstieg. Anschließend wurde gekühlt und entspannt. Zur vollständigen Auspolymerisation wurden nun 6.5 ml 10 %ige wäßrige tert.-Butylhydroperoxidlösung und 6.5 ml 10 %ige wäßrige Hydroxymethansulfinsäurelösung zugegeben.
Es resultierte ein Ethylen-Vinylacetat-Copolymerisat mit einem Ethylen-Gehalt von 15.0 Gew%, einem Vinylacetat-Gehalt von 84.6 Gew% und 0.4 Gew% AMPS. Die Dispersion enthielt kaum Grobanteil, und hatte einen Festgehalt von 53.2 Gew.%, einen pH-Wert von 3.4 und eine mittlere Teilchengröße von 330 nm.

### Herstellung der Dispersionspulver:

### Beispiel 23:

4000 Gew.-Teile Dispersion aus Beispiel 5 und 244.9 Gew.-Teile Phenolsulfonsäure-Formaldehyd-Kondensat als 51.0 %ige Lösung in Wasser (7.0 Gew% bezogen auf Dispersionsfestgehalt) wurden gründlich gemischt. Die Mischung wurde durch eine Zweistoffdüse versprüht. Als Verdüsungskomponente diente auf 4 bar vorgepreßte Luft; die gebildeten Tropfen wurden mit auf 95°C erhitzter Luft im Gleichstrom getrocknet. Das erhaltene Pulver wurde mit 10.0 Gew%, bezogen auf das Gesamtgewicht an polymeren Bestandteilen, handelsüblichem Antiblockmittel (Gemisch aus Calcium-Magnesium-Carbonat und Magnesiumhydrosilikat) versetzt.

### Beispiel 24:

4000 Gew.-Teile Dispersion aus Beispiel 5 und 426.7 Gew.-Teile Naphthalinsulfonsäure-Formaldehyd-Kondensat als 30.0 %ige Lösung in Wasser (7.0 Gew% bezogen auf Dispersionsfestgehalt) wurden gründlich gemischt. Die Mischung wurde durch eine Zweistoffdüse versprüht. Als Verdüsungskomponente diente auf 4 bar vorgepreßte Luft; die gebildeten Tropfen wurden mit auf 80°C erhitzter Luft im Gleichstrom getrocknet. Das erhaltene Pulver wurde mit 10.0 Gew%, bezogen auf das Gesamtgewicht an polymeren Bestandteilen, handelsüblichem Antiblockmittel (Gemisch aus Calcium-Magnesium-Carbonat und Magnesiumhydrosilikat) versetzt.

### Beispiel 25:

4000 Gew.-Teile Dispersion aus Beispiel 5 und 609.3 Gew.-Teile Naphthalinsulfonsäure-Formaldehyd-Kondensat als 30.0 %ige Lösung in Wasser (10.0 Gew% bezogen auf Dispersionsfestgehalt) wurden gründlich gemischt. Die Mischung wurde durch eine Zweistoffdüse versprüht. Als Verdüsungskomponente diente auf 4 bar vorgepreßte Luft; die gebildeten Tropfen wurden mit auf 80°C erhitzter Luft im Gleichstrom getrocknet. Das erhaltene Pulver wurde mit 5.0 Gew%, bezogen auf das Gesamtgewicht an polymeren Bestandteilen, handelsüblichem Antiblockmittel (Gemisch aus Calcium-Magnesium-Carbonat und Magnesiumhydrosilikat) versetzt.

### Vergleichsbeispiel 3:

4000 Gew.-Teile Dispersion aus Vergleichsbeispiel 2 und 664.7 Gew.-Teile Phenolsulfonsäure-Formaldehyd-Kondensat als 43.6 %ige Lösung in Wasser (15.0 Gew% bezogen auf Dispersionsfestgehalt) wurden gründlich gemischt. Die Mischung wurde durch eine Zweistoffdüse versprüht. Als Verdüsungskomponente diente auf 4 bar vorgepreßte Luft; die gebildeten Tropfen wurden mit auf 80°C erhitzter Luft im Gleichstrom getrocknet. Das erhaltene Pulver wurde mit 5 Gew%, bezogen auf das Gesamtgewicht an polymeren Bestandteilen, handelsüblichem Antiblockmittel (Gemisch aus Calcium-Magnesium-Carbonat und Magnesiumhydrosilikat) versetzt.

### Anwendungstechnische Untersuchungen:

Bei den Versuchen zur Ermittlung von Biegezug-, Druck- und Haftzugfestigkeiten wurde ein DIN-Mörtel nach DIN 1164 eingesetzt. Bei allen Versuchen wurde mit einem Kunststoff/Zement-Wert von K/Z = 0.15 (K/Z = 0.15 bedeutet 15 Gew% Dispersionspulver auf eingesetzte Zementmenge) gearbeitet.

| Rezeptur des DIN-Mörtel nach DIN-1164: | |
|---|---|
| Portlandzement PZ-35F | 900 g |
| Normsand (= 2 Beutel) | 2700 g |
| Silicon-Entschäumer S-860 (Fa. Wacker Chemie) | 7.2 g |
| Dispersionspulver | 135 g |
| Wasser | 360 g |

Die pulverförmigen Rezepturbestandteile wurden zusammen mit den Dispersionspulvern aus den Beispielen oder dem Vergleichsbeispiel zu einem Trockenmörtel vermischt. Der Trockenmörtel wurde anschließend mit Wasser auf den entsprechenden Wasser/Zement-Wert (W/Z) von 0.40 (Mörtel ohne Pulverzusatz sowie Vergleichsbeispiel 3 nur mit W/Z = 0.45 möglich) eingestellt und angemischt.

Die Prüfung der Rohmörteleigenschaften zeigt die stark wassereinsparende bzw. verflüssigend wirkenden Eigenschaften der erfindungsgemäßen Dispersionspulver (Beispiele 23, 24, 25). Die Ergebnisse sind in Tabelle 1 zusammengefaßt.

**Tabelle 1:**

| Rohmörteldaten | | | | |
|---|---|---|---|---|
| Dispersionspulver | K/Z | Luftgehalt (%) | Ausbreitmaß ohne Rütteln (cm) | Ausbreitmaß nach Rütteln (cm) |
| ohne Disp.pulver | 0.45 | 3.7 | 10.0 | 13.0 |
| Beispiel 23 | 0.40 | 1.5 | 14.5 | 20.5 |
| Beispiel 24 | 0.40 | 4.3 | 10.0 | 16.0 |
| Beispiel 25 | 0.40 | 4.0 | 11.5 | 18.0 |
| Vergl.beispiel 3 | 0.45 | 4.2 | 10.0 | 16.5 |

Für die Prüfung von Biegezugfestigkeit und Druckfestigkeit wurden Mörtelprismen mit den Maßen 160 x 40 x 40 mm³ nach DIN 1164 hergestellt. Die Ausschalung der Prüfkörper erfolgte jeweils zwei Tage nach dem Füllen der Schalung. Die Schalung wurde während dieser Zeit abgedeckt.

Die Ergebnisse der Biegezug- und Druckfestigkeitsprüfungen sind in Tabelle 2 zusammengefaßt. Die Ergebnisse zeigen eine deutliche Erhöhung der Biegezugfestigkeit durch den Zusatz der erfindungsgemäßen Dispersionspulver im Vergleich zum Mörtel ohne Dispersionspulverzusatz bzw. zum Vergleichsbeispiel 3. Im Gegensatz zum Pulver aus Vergleichsbeispiel 3 wird die Druckfestigkeit der Mörtelprismen durch den Zusatz der erfindungsgemäßen Dispersionspulver nur geringfügig beeinflußt.

**Tabelle 2:**

| Biegezug-/Druckfestigkeiten nach 28 Tagen Lagerung im Normklima (23°C, 50 % relative Feuchte) | | |
|---|---|---|
| Dispersionspulver | Biegezugfestigkeit (N/mm²) | Druckfestigkeit (N/mn²) |
| ohne Disp.pulver | 7.88±0.35 | 47.2±2.2 |
| Beispiel 23 | 13.41±0.44 | 49.8±1.1 |
| Beispiel 24 | 12.13±1.10 | 46.1±0.7 |
| Beispiel 25 | 12.81±0.53 | 47.3±1.2 |
| Vergl.beispiel 3 | 8.92±0.24 | 39.8±1.0 |

Für die Prüfung der Haftzugfestigkeit wurden die Mörtel mit einer Traufel unter Verwendung einer Schablone in 4 mm Schichtstärke auf im Normklima (23°C, 50 % relative Luftfeuchtigkeit) gelagerte Betongehwegplatten (B550, 40 x 40 cm²) aufgezogen. Die Platten wurden im Normklima gelagert. Einen Tag vor dem Prüftermin wurden pro Platte 6 Probekörper mit einem Kernbohrer ausgebohrt und darauf runde Abzugskrampen (Durchmesser 55 mm, Dicke 10 mm) mit einem Zweikomponentenkleber aufgeklebt. Abgezogen wurde mit einem Abzugsgerät mit einer Laststeigerungsrate von 250 N/sec.

Die Ergebnisse der Haftzugfestigkeitsprüfungen sind in Tabelle 3 zusammengefaßt. Die Ergebnisse dieser Prüfung zeigen eine deutliche Erhöhung der Haftzugfestigkeit beim Einsatz der erfindungsgemäßen Dispersionspulver (Beispiele 23, 24, 25) im Vergleich zum Einsatz des Pulvers aus Vergleichsbeispiel 3 und dem Mörtel ohne Dispersionspulverzusatz.

**Tabelle 3:**

| Haftzugfestigkeiten nach 28 Tagen Lagerung im Normklima (23°C, 50 % relative Feuchte) | |
|---|---|
| Dispersionspulver | Haftzugfestigkeit (N/mm²) |
| ohne Dispersionspulver | 1.38±0.11 |
| Beispiel 23 | 3.06±0.17 |
| Beispiel 24 | 2.83±0.15 |
| Beispiel 25 | 3.19±0.16 |
| Vergl.beispiel 3 | 1.81±0.14 |

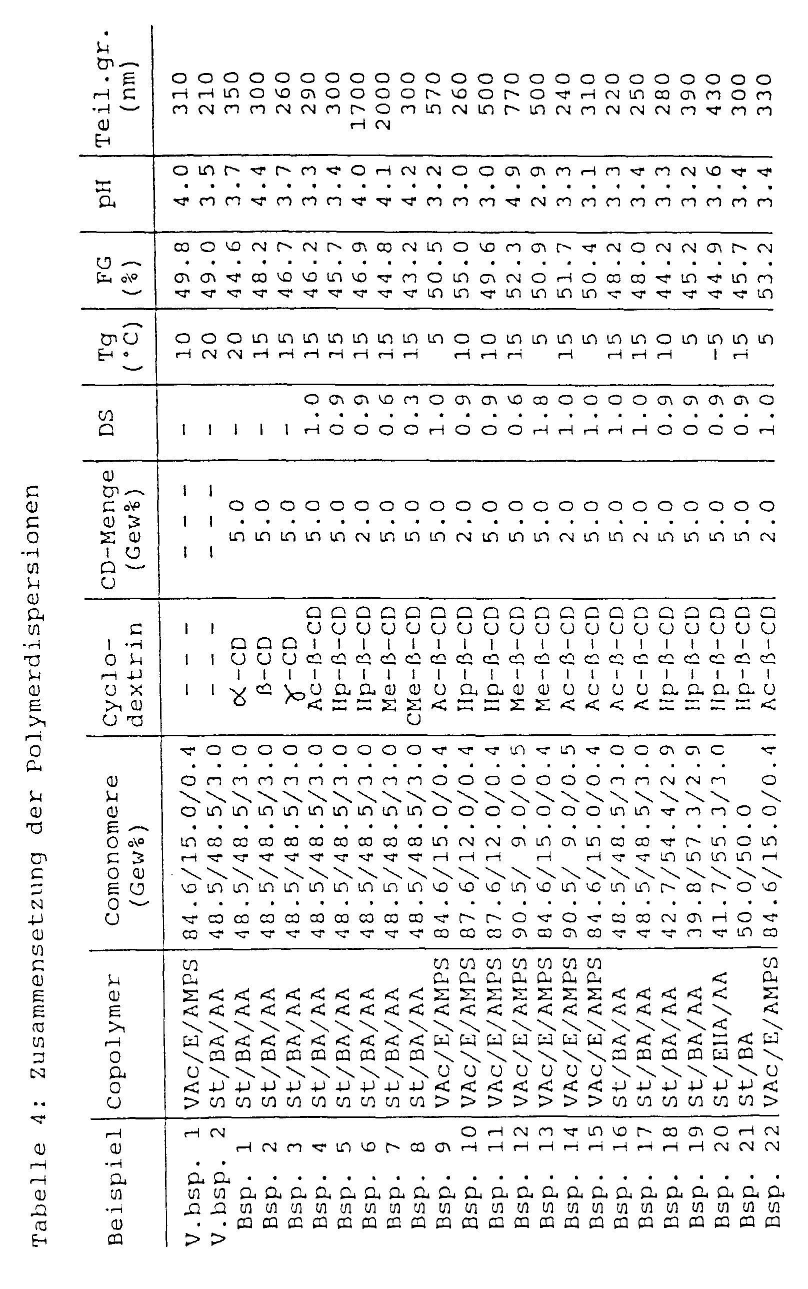

## Patentansprüche

1. Redispergierbare Polymerpulver-Zusammensetzungen, welche Homo- oder Copolymerisate ethylenisch ungesättigter Monomere und Cyclodextrine oder Cyclodextrin-Derivate der allgemeinen Formel 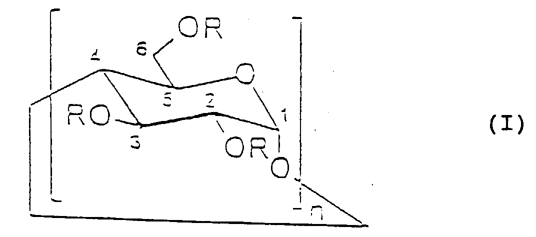 enthalten, wobei n = 6, 7 oder 8 und R gleich oder verschieden ist und die Bedeutung H oder R¹ hat, wobei R¹ gleich oder verschieden ist und die Bedeutung von gegebenenfalls substituierten C₁-C₄-Alkyl-, Hydroxy-C₁-C₄-Alkyl-, Carboxy-C₁-C₄-Alkyl, C₂-C₄-Carboxyl-Reste hat.

2. Redispergierbare Polymerpulver-Zusammensetzungen nach Anspruch 1, dadurch gekennzeichnet, daß Homo- oder Copolymerisate enthaltend ein oder mehrere Monomereinheiten aus der Gruppe der Vinylester von unverzweigten oder verzweigten Carbonsäuren mit 1 bis 18 C-Atomen, aus der Gruppe der Ester der Acrylsäure und Methacrylsäure mit unverzweigten oder verzweigten Alkoholen mit 1 bis 18 C-Atomen, aus der Gruppe der Vinylaromaten, aus der Gruppe der Vinylhalogenide und aus der Gruppe der Olefine enthalten sind.

3. Redispergierbare Polymerpulver-Zusammensetzungen nach Anspruch 1 oder 2, dadurch gekennzeichnet, daß als Cyclodextrin α-Cyclodextrin (Cyclohexaamylose, Formel I: n=6, R=H)), β-Cyclodextrin (Cycloheptaamylose, Formel I: n=7, R=H), γ-Cyclodextrin (Cyclooctaamylose, Formel I: n=8, R=H) oder ein Gemisch der genannten Cyclodextrine enthalten ist.

4. Redispergierbare Polymerpulver-Zusammensetzungen nach Anspruch 1 oder 2, dadurch gekennzeichnet, daß als Cyclodextrin-Derivate ein oder mehrere aus der Gruppe der Methylether, Ethylether oder Propylether von α-, β-, γ-Cyclodextrin; aus der Gruppe der Hydroxyethyl-, Hydroxypropyl- und Dihydroxypropyl-Ether von α-, β- und γ-Cyclodextrin; aus der Gruppe der Carboxymethyl- und Carboxypropylether von α-, β- und γ-Cyclodextrin; aus der Gruppe der Mischether von α-, β- und γ-Cyclodextrin, welche mindestens zwei unterschiedliche Gruppen der genannten Alkylether-, Hydroxyalkylether- oder Carboxyalkylether-Gruppen aufweisen; aus der Gruppe der Essigsäure- (Acetyl-Cyclodextrine) und Propionsäure-Ester (Propionyl-Cyclodextrine) von α-, β- und γ-Cyclodextrin; oder 2-Aminoethyl- oder 2-Chloracetyl-Cyclodextrine enthalten sind.

5. Redispergierbare Polymerpulver-Zusammensetzungen nach Anspruch 1, 2 oder 4, dadurch gekennzeichnet daß der der durch schnittliche Substitutionsgrad (DS-Wert) der Cyclodextrinderivate von 0.5 bis 2.5 beträgt.

6. Redispergierbare Polymerpulver-Zusammensetzungen nach Anspruch 1 bis 5, dadurch gekennzeichnet, daß die Polymerpulver-Zusammensetzungen 0.3 bis 20 Gew% Cyclodextrin oder Cyclodextrin-Derivate, bezogen auf den Polymeranteil, enthalten.

7. Verfahren zur Herstellung von redispergierbaren Polymerpulver-Zusammensetzungen, welche Homo- oder Copolymerisate ethylenisch ungesättigter Monomere und Cyclodextrin oder Cyclodextrinderivate der allgemeinen Formel (I) enthalten, durch
a) Emulsionspolymerisation eines oder mehrerer ethylenisch ungesättigter Monomere mittels Radikalinitiatoren in wäßrigem Medium, in Gegenwart von Cyclodextrin oder Cyclodextrinderivaten der allgemeinen Formel (I), gegebenenfalls von Emulgator, und in Abwesenheit von Schutzkolloiden und
b) Trocknung der dabei erhaltenen wäßrigen Polymerdispersion, gegebenenfalls unter Zugabe von Antiblockmittel und/oder weiterer Zusatzstoffe.

8. Wäßrige Polymerdispersionen mit einem Festgehalt von 30 bis 75 Gew%, welche Homo- oder Copolymerisate ethylenisch ungesättigter Monomere und Cyclodextrin oder Cyclodextrinderivate der allgemeinen Formel (I) enthalten, erhältlich durch Emulsionspolymerisation eines oder mehrerer ethylenisch ungesättigter Monomere mittels Radikalinitiatoren in wäßrigem Medium, in Gegenwart von Cyclodextrin oder Cyclodextrinderivaten der allgemeinen Formel (I), gegebenenfalls von Emulgator, und in Abwesenheit von Schutzkolloiden.

9. Verwendung von redispergierbaren Polymerpulver-Zusammensetzungen nach Anspruch 1 bis 6 als Zusätze zu hydraulischen Bindemitteln wie Zement und Gips und als Zusätze zu Beton, Bauklebern, Mörteln, Spachtelmassen und Verlaufsmassen.

10. Verwendung von redispergierbaren Polymerpulver-Zusammensetzungen nach Anspruch 1 bis 6 als Bindemittel für Beschichtungen, Putze und Anstriche, insbesonders Anstrichfarben.

11. Verwendung von redispergierbaren Polymerpulver-Zusammensetzungen nach Anspruch 1 bis 6 als Klebstoffe für Holz, Papier und Textilien.

12. Verwendung von redispergierbaren Polymerpulver-Zusammensetzungen nach Anspruch 1 bis 6 als Bindemittel bei der Papierherstellung.

13. Verwendung von redispergierbaren Polymerpulver-Zusammensetzungen nach Anspruch 1 bis 6 als Bindemittel für Preßmassen und Formkörper.

14. Verwendung von redispergierbaren Polymerpulver-Zusammensetzungen nach Anspruch 1 bis 6 als Bindemittel zur Herstellung von Wirkstoffe enthaltenden, bevorzugt durch Direktverpressung hergestellten, Preßkörpern.

## Claims

1. Redispersible polymer powder compositions which comprise a homo- or copolymer of ethylenically unsaturated monomers and a cyclodextrin or cyclodextrin derivative of the general formula 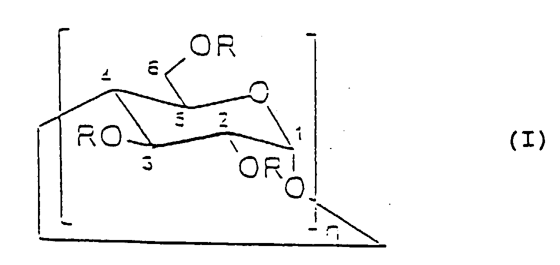 in which n = 6, 7 or 8 and R is identical or different and has the meaning of H or R¹, where R¹ is identical or different and has the meaning of optionally substituted C₁-C₄-alkyl, hydroxy-C₁-C₄-alkyl, carboxy-C₁-C₄-alkyl or C₂-C₄-carboxyl radicals.

2. Redispersible polymer powder compositions according to Claim 1, characterized in that there are contained homo- or copolymers comprising one or more monomer units from the group consisting of vinyl esters of unbranched or branched carboxylic acids having 1 to 18 C atoms, from the group consisting of esters of acrylic acid and methacrylic acid with unbranched or branched alcohols having 1 to 18 C atoms, from the group consisting of vinylaromatics, from the group consisting of vinyl halides and from the group consisting of olefins.

3. Redispersible polymer powder compositions according to Claim 1 or 2, characterized in that there is contained as the cyclodextrin α-cyclodextrin (cyclohexaamylose, formula I: n=6, R=H), β-cyclodextrin (cycloheptaamylose, formula I: n=7, R=H), γ-cyclodextrin (cyclooctaamylose, formula I: n=8, R=H) or a mixture of the cyclodextrins mentioned.

4. Redispersible polymer powder compositions according to Claim 1 or 2, characterized in that there is contained as the cyclodextrin derivative one or more derivatives from the group consisting of the methyl ethers, ethyl ethers or propyl ethers of α-, β- and γ-cyclodextrin; from the group consisting of the hydroxyethyl, hydroxypropyl and dihydroxypropyl ethers of α-, β-and γ-cyclodextrin; from the group consisting of the carboxymethyl and carboxypropyl ethers of *α-,* β- and γ-cyclodextrin; from the group consisting of the mixed ethers of α-, β- and γ-cyclodextrin which contain at least two different groups of the alkyl ether, hydroxyalkyl ether or carboxyalkyl ether groups mentioned; from the group consisting of the acetic acid esters (acetyl-cyclodextrins) and propionic acid esters (propionyl-cyclodextrins) of *α-,* β- and γ-cyclodextrin; or 2-aminoethyl- or 2-chloroacetyl-cyclodextrin.

5. Redispersible polymer powder compositions according to Claim 1, 2 or 4, characterized in that the average degree of substitution (DS value) of the cyclodextrin derivatives is 0.5 to 2.5.

6. Redispersible polymer powder compqsitions according to Claims 1 to 5, characterized in that the polymer powder composition comprises 0.3 to 20% by weight of cyclodextrin or cyclodextrin derivatives, based on the polymer content.

7. Process for the preparation of redispersible polymer powder compositions which comprise homo- or copolymers of ethylenically unsaturated monomers and cyclodextrin or cyclodextrin derivatives of the general formula I by
a) emulsion polymerization of one or more ethylenically unsaturated monomers by means of free radical initiators in an aqueous medium in the presence of cyclodextrin or a cyclodextrin derivative of the general formula (I) and if appropriate emulsifier and in the absence of protective colloids and
b) drying of the aqueous polymer dispersion obtained by this procedure, if appropriate with the addition of antiblocking agents and/or further additives.

8. Aqueous polymer dispersions having a solids content of 30 to 75% by weight which comprise homo- or copolymers of ethylenically unsaturated monomers and cyclodextrin or cyclodextrin derivatives of the general formula (I), obtainable by emulsion polymerization of one or more ethylenically unsaturated monomers by means of free radical initiators in an aqueous medium in the presence of cyclodextrin or a cyclodextrin derivative of the general formula (I) and if appropriate emulsifier and in the absence of a protective colloids.

9. Use of redispersible polymer powder compositions according to Claims 1 to 6 as additives to hydraulic binders, such as cement and gypsum, and as an additives to concrete, building adhesives, mortars, filling compositions and flow control compositions.

10. Use of redispersible polymer powder compositions according to Claims 1 to 6 as binders for coatings, plasters and coverings, in particular paints.

11. Use of redispersible polymer powder compositions according to Claims 1 to 6 as adhesives for wood, paper and textiles.

12. Use of redispersible polymer powder compositions according to Claims 1 to 6 as binders in papermaking.

13. Use of redispersible polymer powder compositions according to Claims 1 to 6 as binders for compression moulding compositions and shaped articles.

14. Use of redispersible polymer powder compositions according to Claims 1 to 6 as binders for the production of compression moulded articles which comprise active compounds and are preferably produced by direct pressing.

## Revendications

1. Compositions redispersables de poudre de polymère, qui contiennent des homo- ou copolymères de monomères éthyléniquement insaturés et des cyclodextrines ou dérivés de cyclodextrines de formule générale : 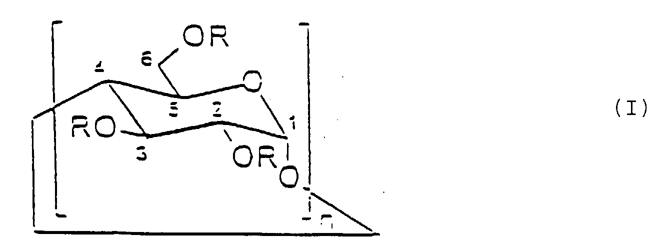 dans laquelle :
n = 6, 7 ou 8, et
R est identique ou différent et signifie H ou R¹,
R¹ étant identique ou différent et signifiant des radicaux, le cas échéant substitués, alcoyle en C₁-C₄, hydroxyalcoyle en C₁-C₄, carboxyalcoyle en C₁-C₄, carboxyle en C₂-C₄.

2. Compositions redispersables de poudre de polymère suivant la revendication 1, caractérisées en ce qu'elles sont composées d'homo- ou copolymères contenant une ou plusieurs unités monomères du groupe des esters vinyliques d'acides carboxyliques linéaires ou ramifiés ayant 1 à 18 atomes de carbone, du groupe des esters de l'acide acrylique et de l'acide méthacrylique avec des alcools linéaires ou ramifiés ayant 1 à 18 atomes de carbone, du groupe des vinylaromatiques, du groupe des halogénures de vinyle et du groupe des oléfines.

3. Compositions redispersables de poudre de polymère suivant la revendication 1 ou 2, caractérisées en ce qu'elles contiennent comme cyclodextrine l'α-cyclodextrine (cyclohexaamylose, formule I : n=6, R=H), la β-cyclodextrine (cycloheptaamylose, formule I : n=7, R=H), la γ-cyclodextrine (cyclooctaamylose, formule I : n=8, R=H) ou un mélange des cyclodextrines citées.

4. Compositions redispersables de poudre de polymère suivant la revendication 1 ou 2, caractérisées en ce qu'elles contiennent comme dérivés de cyclodextrine un ou plusieurs dérivés du groupe des éthers méthyliques, éther éthyliques ou éther propyliques de l'α-, β- ou γ-cyclodextrine; du groupe des éthers hydroxyéthyliques, hydroxypropyliques et dihydroxypropyliques de l'α-, β- ou γ-cyclodextrine; du groupe des éthers carboxyméthyliques et carboxypropyliques de l'α-, β- et γ-cyclodextrine; du groupe des éthers mixtes de l'α-, β- ou γ-cyclodextrine, qui présentent au moins deux groupes différents des groupes éthers alcoyliques, éthers hydroxyalcoyliques ou éthers carboxyalcoyliques cités; du groupe des esters d'acide acétique (acétylcyclodextrines) et d'acide propionique (propionylcyclodextrines) de l'α-, β- et γ-cyclodextrine; ou les 2-aminoéthyl- ou 2-chloroacétylcyclodextrines.

5. Compositions redispersables de poudre de polymère suivant la revendication 1, 2 ou 4, caractérisées en ce que le degré de substitution moyen (DS) des dérivés de cyclodextrine vaut de 0,5 à 2,5.

6. Compositions redispersables de poudre de polymère suivant les revendications 1 à 5, caractérisées en ce que les compositions de poudre de polymère contiennent 0,3 à 20% en poids de cyclodextrine ou de dérivés de cyclodextrine par rapport à la fraction des polymères.

7. Procédé de préparation de compositions redispersables de poudre de polymère, qui contiennent des homo- ou des copolymères de monomères éthyléniquement insaturés et de la cyclodextrine ou des dérivés de cyclodextrine de formule générale (I), par
a) polymérisation en émulsion d'un ou plusieurs monomères éthyléniquement insaturés, au moyen d'initiateurs radicalaires en milieu aqueux, en présence de cyclodextrine ou de dérivés de cyclodextrine de formule générale (I), le cas échéant, d'un émulsifiant, et en l'absence de colloïdes protecteurs, et
b) séchage de la dispersion aqueuse de polymère ainsi obtenue, le cas échéant avec addition d'agents antibloquants et/ou d'autres additifs.

8. Dispersions aqueuses de polymères ayant une teneur en matières solides de 30 à 75% en poids, qui contiennent des homo- ou des copolymères de monomères éthyléniquement insaturés et de la cyclodextrine ou des dérivés de cyclodextrine de formule générale (I), pouvant être obtenues par polymérisation en émulsion d'un ou plusieurs monomères éthyléniquement insaturés au moyen d'initiateurs radicalaires en milieu aqueux, en présence de cyclodextrine ou de dérivés de cyclodextrine de formule générale (I), le cas échéant d'un émulsifiant, et en l'absence de colloïdes protecteurs.

9. Utilisation de compositions redispersables de poudre de polymère suivant les revendications 1 à 6 comme additifs pour des liants hydrauliques comme le ciment ou le plâtre, et comme additifs pour le béton, les colles pour bâtiments, les mortiers, les enduits et les agents d'égalisation.

10. Utilisation de compositions redispersables de poudre de polymère suivant les revendications 1 à 6 comme liants pour revêtements, enduits et crépis, en particulier les peintures.

11. Utilisation de compositions redispersables de poudre de polymère suivant les revendications 1 à 6 comme colles pour le bois, le papier et les textiles.

12. Utilisation de compositions redispersables de poudre de polymère suivant les revendications 1 à 6 comme liants dans la fabrication du papier.

13. Utilisation de compositions redispersables de poudre de polymère suivant les revendications 1 à 6 comme liants pour matières à mouler et corps moulés.

14. Utilisation de compositions redispersables de poudre de polymère suivant les revendications 1 à 6 comme liants pour la fabrication de corps moulés, de préférence préparés par compression directe, contenant des agents actifs.
